# EUROPÄISCHE PATENTSCHRIFT

(11) **EP 4 373 456 B1**
(45) Veröffentlichungstag und Bekanntmachung des Hinweises auf die Patenterteilung: **26.11.2025**
(21) Anmeldenummer: 22754400.4
(22) Anmeldetag: 22.07.2022
(51) Int. Cl.: A61J 1/14, B65D 1/00, C03C 17/30, B65D 23/02, A61K 36/00, B65D 25/14, B65D 81/24, C03C 17/00

(54) **VERWENDUNG VON SILIKONISIERTEN GLASBEHÄLTERN FÜR FLÜSSIGE PFLANZENEXTRAKTE**
USE OF SILICONIZED GLASS CONTAINERS FOR LIQUID PLANT EXTRACTS
UTILISATION DE RÉCIPIENTS EN VERRE SILICONISÉ POUR LES EXTRAITS LIQUIDES DE PLANTES

(30) Priorität: 23.07.2021 EP 21187445
(43) Veröffentlichungstag der Anmeldung: 29.05.2024
(73) Patentinhaber: Bionorica SE, 92318 Neumarkt (DE)
(72) Erfinder: HABLON, Ilona, 92367 Pilsach (DE); FERSTL, Matthias, 92331 Parsberg (DE); REHER, Markus, 61440 Oberursel (DE)
(74) Vertreter: Simandi, Claus
(86) Internationale Anmeldenummer: PCT/EP2022/070701
(87) Internationale Veröffentlichungsnummer: WO 2023/002049

(56) Entgegenhaltungen:
- EP-A1- 2 857 371
- EP-A1- 3 260 431
- WO-A1-94/00397
- WO-A2-01/66089
- GRAUWILER ET AL: "Development of a LC/MS/MS method for the analysis of cannabinoids in human EDTA-plasma and urine after small doses of Cannabis sativa extracts", JOURNAL OF CHROMATOGRAPHY B, ELSEVIER, AMSTERDAM, NL, vol. 850, no. 1-2, 24 April 2007 (2007-04-24), pages 515 - 522, XP022044102, ISSN: 1570-0232, DOI: 10.1016/J.JCHROMB.2006.12.045
- THOMPSON ROBERT Q ET AL: "Chemical comparison ofPrunus africanabark and pygeum products marketed for prostate health", JOURNAL OF PHARMACEUTICAL AND BIOMEDICAL ANALYSIS, ELSEVIER B.V, AMSTERDAM, NL, vol. 163, 5 October 2018 (2018-10-05), pages 162 - 169, XP085515571, ISSN: 0731-7085, DOI: 10.1016/J.JPBA.2018.10.004
- RATOLA N ET AL: "Microwave-assisted extraction and ultrasonic extraction to determine polycyclic aromatic hydrocarbons in needles and bark of Pinus pinaster Ait. and Pinus pinea L. by GC-MS", TALANTA, ELSEVIER, AMSTERDAM, NL, vol. 77, no. 3, 15 January 2009 (2009-01-15), pages 1120 - 1128, XP025760514, ISSN: 0039-9140, [retrieved on 20080826], DOI: 10.1016/J.TALANTA.2008.08.010

## Beschreibung

Die vorliegende Erfindung betrifft die Verwendung eines verschließbaren Glasbehälters zur Aufbewahrung von flüssigen Pflanzenextrakten, wobei die Glasinnenwand silikonisiert ist.

Arzneipflanzen enthalten, ggfs. in bestimmten Pflanzenteilen wie Wurzeln, Blättern, Blüten oder Früchten angereichert, Inhaltsstoffe mit pharmakologischer Wirkung und bilden die Grundlage für eine beachtliche Anzahl von Mitteln, wie Arzneimittel und Nahrungsergänzungsmittel. Zur Gewinnung dieser Inhaltsstoffe gibt es unterschiedliche Verfahren, von denen die meisten nach dem Prinzip einer wie auch immer gearteten Extraktion einschließlich Mazeration oder Perkolation der Pflanzen mit einem geeigneten Extraktionsmittel bzw. Lösungsmittel arbeiten, zumeist jedoch Alkohol oder Wasser-Alkohol und infolgedessen eine mehr oder weniger selektive Lösung und Anreicherung bestimmter pflanzlicher Wirkstoffe oder Wirkstoffgruppen in dem Extraktionsmittel bzw. Auszug ergeben. Gemäß WO 2021/019097 A1 der Anmelderin können mittels Membranfitration alkohol-freie flüssige Pflanzenextrakte bereitgestellt werden.

Solche Pflanzenextrakte können flüssig, halbfest oder fest und ein Trockenextrakt (Extracta sicca) sein, wobei z.B. der Extraktionsrückstand, der erhaltene Fluidextrakt (Extracta fluida) als Überstand bzw. die erhaltene Tinktur (Tincturae) zur Trockene eingeengt wird. Eine Trocknung kann z.B. mittels Wirbelschichttrocknung oder mittels Einengung zu einem Dick- oder Spissumextrakt (zähflüssiger Extrakt, Extracta spissa) erfolgen mit anschließender Vakuumbandtrocknung oder Hordentrocknung, siehe auch z.B. EP 0 753 306 B1 der Anmelderin.

### Schaubild:

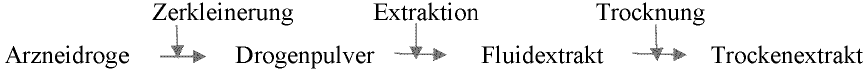

Es werden im Rahmen dieser Erfindung sogenannte flüssige Pflanzenextrakte betrachtet.

Selbstverständlich können ebenfalls die genannten Trockenextrakte wieder in einen flüssigen Pflanzenextrakt überführt werden.

So genannte standardisierte Pflanzenextrakte werden innerhalb zulässiger Grenzen auf einen vorgegebenen Gehalt an wirksamkeitsbestimmenden Inhaltsstoffen (Leitsubstanzen) eingestellt. Die Einstellung kann durch Mischen von Extraktchargen erfolgen und / oder durch Zugabe von Hilfsstoffen (siehe z.B. Ph. Eur. Monographien).

So genannte quantifizierte Pflanzenextrakte werden auf einen definierten Bereich von wirksamkeitsmitbestimmenden Inhaltsstoffen (Leitsubstanzen) eingestellt. Die Einstellung kann durch Mischen von Extraktchargen erfolgen.

Zumeist werden sogenannte Fluidextrakte als flüssige Pflanzenextrakte in galenische Formen, wie Tropfen und Säfte eingebracht.

Beispielsweise vertreibt die Anmelderin Pflanzenextrakte in der galenischen Form von Tropfen und Säfte der Marken Imupret^{®}, Sinupret^{®}, Canephron^{®} oder Bronchipret^{®}.

Flüssige Pflanzenextrakte sind jedoch hinsichtlich der Stabilität eine Herausforderung. Die physikalische und chemische Instabilität sowie die Empfindlichkeit für mikrobiologische Veränderungen dieser Pflanzenextrakte ist allgemein bekannt. Diese Instabilität kann zu Trübungen, Ausfällungen, Farbänderung, pH-Wert-Änderung und zu unterschiedlichen Abbauprozessen der enthaltenen Komponenten führen. Dadurch kann die Haltbarkeit bzw. Stabilität der flüssigen Pflanzenextrakte, insbesondere bei langer Aufbewahrung eingeschränkt sein.

In der pharmazeutischen Technologie wird die Stabilität von flüssigen pflanzlichen Arzneimitteln zumeist durch spezielle technologische Schritte (z. B. Filtration, Entkeimung, Begasung mit Schutzatmosphäre) und durch sorgfältige Auswahl einer Galenik oder Formulierung (z. B. Hilfsstoffe für Erhöhung der Löslichkeit der Komponenten, Erhöhung der Viskosität, Zusatz von Antioxidantien und Konservierungsmitteln, etc.) erreicht. Gleiche Vorgehensweisen findet man im Bereich von Lebensmitteln und Medizinprodukten.

Nach wie vor gilt es solche flüssigen Pflanzenextrakte in einer ausreichenden Stabilität einem Verbraucher zur Verfügung stellen.

Es ist bekannt, dass die Auswahl des Primärpackmittels die Haltbarkeit bzw. Stabilität von flüssigen Pflanzenextrakten beeinflusst. Weitestgehend inerte Gummi- und Kunststoffmaterialien mit strengen Vorgaben bezüglich auslösbarer Bestandteile und Glas sind die am häufigsten eingesetzten Packmaterialien.

Die Glasqualität sollte mindestens Hydrolyseklasse III (siehe Ph. Eur. 3.2.1) entsprechen. In den meisten Fällen wird Braunglas eingesetzt, um zusätzlich Lichtschutz für den Inhalt zu gewährleisten. Bevorzugte Gläser sind zumeist nicht abschließend Neutralglas, insbesondere Natronkalk-Silicatglas.

Die Gläser sind jedoch nicht vollständig inert. Sie können Metallionen abgeben, die in den Formulierungen katalytisch Abbaureaktionen auslösen können. Ebenfalls können Fehlstellen der Oberfläche chemische Reaktionen katalysieren und als Keimstelle für Kristallisation dienen.

Die Silikonisierung von Gläsern, insbesondere Glasbehältern für pharmazeutische Produkte ist im Stand der Technik beschrieben. Zumeist ist eine Silikonisierung erforderlich für Dosiereinrichtungen, deren Funktion durch Bewegung von Teilen des Packmittels ausgelöst werden können (wie z. B. Fertigspritzen). Die Silikonisierung ermöglicht das notwendige Gleiten der Packmittelkomponenten.

Die (Innen-)Silikonschicht kann temporär (Befilmung) oder dauerhaft mittels Einbrennsilikonisierung oder Beschichtungsverfahren, wie die chemische Gasphasenabscheidung (chemical vapour deposition, CVD) auf eine Glasoberfläche aufgebracht werden. Die unterschiedlichen Herstellungsmethoden sind dem Fachmann bekannt (Diss. T. Mundry, Humboldt-Universität, Berlin, 1999).

Die Einbrennsilikonisierung liefert zumeist eine homogene, ca. 15 - 50 nm dünne hydrophobe Schicht und deckt die initial vorhandene Glasoberfläche und vorhandene Fehlstellen ab (Reuter B., Petersen C., Die Silikonisierung von Spritzen, TechnoPharm2, Nr.4, 238-244 (2012)). Infolgedessen wird eine reduzierte Wechselwirkung zwischen Packmittel und Produkt erreicht.

DE 10 2009 021 501 B4 offenbart die Aufbewahrung von med. geeigneten Pflanzenextrakten in Silikonbeuteln [0031, 0018, Patentansprüche 19 und 21]. Allerdings wird ein innensilikonisierter Glasbehälter nicht offenbart, und zudem können solche Silikonbeutel bei längerer Lagerung reißen oder Feuchtigkeit anziehen.

EP 2 857 371 A1 und EP 3 260 431 A1 offenbaren inerte, silanisierte Glasbehälter und deren generelle Verwendung in Medizin und Pharmazie.

WO 94/00397 A1 offenbart die Verwendung einer Silikon-Innenschicht zur Verhinderung des Übertritts von Blei aus dem Glasbehälter in die Flüssigkeit. Die Aufbewahrung alkoholischer Flüssigkeiten wird zwar in der Beschreibung erwähnt, ist aber nirgends in den Beispielen ausgeführt.

WO 01/66089 A2 offenbart Glasbehälter mit einer silikonisierten Innenschicht für die Verabreichung von THC als Aerosol oder Spray.

Grauweiler et al. "Development of a LC/MS/MS method for the analysis of cannabinoids in human EDTA-plasma and urine after small doses of Cannabis sativa extracts", JOURNAL OF CHROMATOGRAPHY B, ELSEVIER, AMSTERDAM, NL,Bd. 850, Nr. 1-2, 24. April 2007, Seiten 515-522, offenbart die Lagerstabilität von Cannabinoiden in Urinproben in silanisierten Vials aus Glas.

Thompson Robert Q. et al. "Chemical comparison of Prunus africanabark and pygeum products marketed for prostate health", JOURNAL OF PHARMACEUTICAL AND BIOMEDICAL ANALYSIS, ELSEVIER B.V, AMSTERDAM, NL, Bd. 163, 5. Oktober 2018, Seiten 162-169, offenbart die Verwendung von silanisierten Vials aus Glas zur Aufbewahrung von Pygeum Rindenextrakten mit Aceton/Ethylacetat als Lösungsmittel.

Ratola N. et al. "Microwave-assisted extraction and ultrasonic extraction to determine polycyclic aromatic hydrocarbons in needles and bark of Pinus pinaster Ait. and Pinus pinea L. by GC-MS", TALANTA, ELSEVIER, AMSTERDAM, NL, Bd. 77, Nr. 3, 15. Januar 2009, Seiten 1120-1128, offenbart die verbesserte Lagerstabilität von Pinus Extrakten in silanisierten Glasvials mit Hexan/Dichlormethan als Lösungsmittel.

Keines der vorgenannten Dokumente offenbart die Verwendung eines innen silanisierten Glasbehälters zur Aufbewahrung eines flüssigen Pflanzenextrakts gemäß Anspruch 1.

Es besteht ein hohes Bedürfnis solche flüssigen Pflanzenextrakte mit verbesserter Stabilität bereitzustellen. Daher besteht die Aufgabe die Stabilität von flüssigen Pflanzenextrakten zu verbessern, insbesondere deren Aufbewahrung, insbesondere über lange Zeiträume, wie Monate und Jahre, so dass insbesondere die Wirksamkeit als Arzneimittel oder Nahrungsergänzungsmittel der flüssigen Pflanzenextrakte erhalten bleibt.

Überraschender Weise konnten die Erfinder feststellen, dass flüssige Pflanzenextrakte, insbesondere als Arzneimittel oder Nahrungsergänzungsmittel, in Gegenwart oder Kontakt von silikonisiertem Glas als Packmittel im Fall der Aufbewahrung eine deutliche Verbesserung der physikalischen Stabilität, insbesondere sowohl eine deutliche Reduktion der Trübung oder Ausfällung als auch pH-Stabilität als auch eine Verzögerung der chemischen Abbaureaktionen erreichen.

Daher betrifft die Erfindung die Verwendung von einem verschließbaren, silikonisierten Glasbehälter zur Aufbewahrung von mindestens einem flüssigen Pflanzenextrakt, wobei der verschließbare, silikonisierte Glasbehälter eine Innenwand mit einer Silikonschicht aufweist, wobei der Pflanzenextrakt i.) in einem oder mehreren Lösungsmitteln gelöst vorliegt, ausgewählt aus der Gruppe alkoholhaltige Lösungsmittel, C1-C5-Alkohole, Ethanol, Wasser, Gemisch aus Wasser / Ethanol, oder ein entalkoholisierter flüssiger Pflanzenextrakt davon und Polyphenole enthält,
ii.) wobei der Pflanzenextrakt ein Arzneimittel ist, und weitere Hilfs- und Zusatzstoffe aufweist,
iii.) wobei das Extraktionsmittel ausgewählt ist aus der Gruppe alkoholhaltige Lösungsmittel, C1-C5-Alkohole, Ethanol, Wasser, Gemisch aus Wasser / Ethanol..

Im Rahmen dieser Erfindung ist ein silikonisierter Glasbehälter ein solcher, dessen Innenwand eine Silikonschicht aufweist. Der Begriff Innenwand umfasst ebenfalls einen Boden, ggfs. samt Öffnung. Beispielsweise kann eine Silikonschicht mittels einem Alkylchlorsilan - Gemisch auf das Glas aufgebracht werden. Weiterhin können Silikonöle eingesetzt werden. Ebenfalls können Silanole, Silandiole oder Silantriole im Gemisch eingesetzt werden.

Der silikonisierte Glasbehälter umfasst erfindungsgemäß keine Spritze, sondern einen verschließbaren Glasbehälter, insbesondere einseitig verschließbar, welcher mittels eines Deckels oder Aufsatzes an einer Öffnung verschlossen werden kann. Ein geeigneter Verschluss ist z.B. ein Drehverschluss, Aufsatz oder Druckaufsatz. Der Aufsatz kann z.B. eine Dosierungseinheit aufweisen. Weiterhin sind solche Glasbehälter erfindungsgemäß umfasst, welche für eine topische, orale, parenterale oder nasale Applikation geeignet sind, und eine entsprechend geeignete Austrittsöffnung aufweisen (z.B. Flasche mit Drehverschluss, Flasche mit Pumpapplikator, Nasenspray, Nasentropfen, etc.). Der silikonisierte Glasbehälter kann mit mindestens einem flüssigen Pflanzenextrakt, ggfs. samt Hilfs- und Zusatzstoffen zur Aufbewahrung oder Lagerung befüllt werden.

Im Rahmen dieser Erfindung wird unter einem "Pflanzenextrakt" ein Vielkomponentengemisch aus Naturstoffen verstanden, welches mehr als zwei Naturstoffe, insbesondere mehr als 10 oder 100 Naturstoffe, insbesondere mehr als 200, 300, 500 oder 1.000 Naturstoffe enthält. Pflanzenextrakte können beispielsweise mittels Extraktion, Perkolation oder Mazeration aus Pflanzenmaterialien gewonnen werden. Als Extraktionsmittel können alkoholhaltige Lösungsmittel, C1-C5-Alkohole, Ethanol verwendet werden. Eine übliche Extraktion ist beispielsweise eine wässrig-ethanolische Extraktion, insbesondere ein Gemisch aus Wasser / Ethanol (50:50 v/v, 70:30 v/v, 30:70 v/v) z.B. bei 15 bis 80 Grad Celsius und Normaldruck. Der Begriff Pflanzenextrakt umfasst insbesondere Fluidextrakte (Extractum fluidum), wobei eine flüssige Drogenzubereitung bereitgestellt wird, bei der für die Extraktion der Droge vorzugsweise möglichst wenig Extraktionsflüssigkeit eingesetzt wird. Dies wirkt sich auf das Drogen-Extrakt-Verhältnis (DEV) aus.

Ein flüssiger Pflanzenextrakt im Sinne dieser Erfindung bedeutet, dass der Pflanzenextrakt zum überwiegenden Teil oder vollständig in einem oder mehreren Lösungsmitteln, ggfs. samt Hilfs- und Zusatzstoffen, gelöst bzw. flüssig vorliegt. Lösungsmittel sind vorzugsweise die Extraktionsmittel, wie alkoholhaltige Lösungsmittel, C1-C5-Alkohole, Ethanol, Wasser, Gemisch aus Wasser / Ethanol , insbesondere 50:50 v/v, 70:30 v/v, 30:70 v/v oder aus solchen Extrakten davon entalkoholisierte flüssige Pflanzenextrakte, vorzugsweise mittels Membranfiltration in einer Ultrafiltration, Nanofiltration oder Umkehrosmose.

Solche flüssigen Pflanzenextrakte weisen charakteristisch sekundäre Pflanzeninhaltsstoffe auf, die für die Stabilität des flüssigen Pflanzenextrakts relevant sind, und Ausfällung bzw. Trübung verursachen. Insbesondere Polyphenole sind zu nennen, solche wie Phenolsäuren, Phenolcarbonsäuren (z.B. Rosmarinsäure), Hydroxybenzoesäuren und Flavonoide (z.B. Luteolin, Rutin, Naringenin, Apigenin, Eriodictyol). Solche Ausfällungen bzw. Trübungen, insbesondere in Form von Partikeln oder Aggregaten, entziehen dem flüssigen Pflanzextrakte potentiellen Wirkstoff, auch durch Einschluss von wertvollen Pflanzeninhaltsstoffen.

Daher betrifft die Erfindung, insbesondere solche flüssigen Pflanzenextrakte, welche Polyphenole enthalten, insbesondere Phenolsäuren, Phenolcarbonsäuren (z.B. Rosmarinsäure), Hydroxybenzoesäuren und Flavonoide.

Die Aufbewahrung des flüssigen Pflanzenextrakts in einem innen-silikonisierten Glasbehälter kann besonders vorteilhaft bei verbesserter Stabilität drei Monate, vorzugsweise 6 Monate oder 12 Monate oder länger erfolgen, da keine oder verminderte Ausfällung bzw. Trübung eintritt im Vergleich zu nicht-innen-silikonisierten Glasbehälter, wie durch die Beispiele belegt, und folglich mehr Wirkstoff in der Flüssigkeit eines Pflanzenextrakts in einem innen-silikonisierten Glasbehälter verbleibt.

Solche flüssigen Pflanzenextrakte können nicht abschließend aus Pflanzenmaterialien erhalten werden, solche wie Achillea, Aloe, Althaea, Angelica, Arnika, Artemisia, Cannabis, Capsicum, Carum, Caulophyllum, Centaurium, Chelidonium, Cimicifuga, Cnicus, Citrus, Crataegus, Cyclamen, Cynara, Echinacea, Equisetum, Glycyrrhiza, Guaiacum, Hedeara, Humulus, Iberis, Iris, Juglans, Lavandula, Levisticum, Lilium, Matricaria, Melissa, Mentha, Basilicum (Ocimum), Passiflora, Pelargonium, Phytolacca, Pimpinella, Primula, Potentilla, Punica, Quercus, Rosmarinus, Rumex, Salix, Salvia, Sambucus, Silybum, Strychnos, Taraxacum, Thymus, Vaccinium, Valeriana, Vebena, Vitex, Vitis.

Solche flüssigen Pflanzenextrakte können nicht abschließend aus Pflanzenmaterialien erhalten werden, solche wie Achillea millefolium Aloe Vera, Althaea officinalis, Angelica archangelica, Arnika montana, Artemisia vulgaris, annua und absinthium, Cannabis indica, Cannabis ruderalis, Cannabis sativa, Capsicum annuum, Carum carvi, Caulophyllum thalictroides, Centaurium, Chelidonium majus, Cimicifuga racemose, Cnicus benedictus Citrus reticulata, Citrus sinensis, Citrus junos, Citrus medica, Citrus maxima, Citrus aurantifolia, Citrus aurantium, Citrus hystrix, Citrus limon, Citrus paradisi, Cistus incanus, Crataegus, Cyclamen purpurascens, Cynara cardunculus, Echinacea purpurea und angust, Equisetum arvense, Glycyrrhiza glabra, Glycyrrhiza inflata, Glycyrrhiza uralensis, Guaiacum officinale und sanctum, Hedeara helix, Humulus lupulus, Iberis amara, Juglans regia, Lavandula angustifolia, Levisticum officinale, Lilium tigrinum, Matricaria chamomilla, Melissa officinalis, Mentha candensis, Mentha arvensis, Mentha piperita, Ocimum basilicum, Passiflora, Phytolacca americana, Pelargonium sidoides, Pimpinella anisum, Primula veris, elatior und vulgaris, Potentilla anserina, Punica granatum, Quercus robur, Quercus petraea, Quercus pubescens, Rosmarinus, Rumex cripus, Rumex obtusifolius, Rumex alpinus, Rumex patientia, Rumex acetosa, Rumex acetosella, Rumex thyrsiflorus, Salix purpurea, Salix daphnoides, Salix fragilis, Salvia officinalis, Sambucus nigra, Silybum marianum, Strychnos ignatii, Taraxacum officinale, Thymus vulgaris, Vaccinium macrocarpon, Vaccinium myrtillus, Valeriana officinalis, Vebena officinalis, Vitex agnus castus, Vitis vinifera.

In einer weiteren Ausführungsform der Erfindung kann eine vorteilhafte Vor- oder Nachbehandlung der Pflanzenextrakte erfolgen, solche wie Entkeimung mittels Kurzzeiterhitzung, Pasteurisierung, Ultrahocherhitzung, entkeimende Filtration, o. ä.. Ebenso ist eine Konservierung mit Konservierungsmitteln, wie z.B. Kaliumsorbat möglich.

Der besagte Pflanzenextrakt wird in flüssiger Form bereitgestellt, insbesondere in einer Arzneiform oder galenischen Form ausgewählt aus der Gruppe flüssiger Zubereitung, Tropfen, Saft, Sirup, Aufguss, insbesondere Rachenspray sowie Desinfektionslösungen, Nasenspray, flüssige Präparate für die Inhalation, Spüllösungen, insbesondere in Kombination mit physiologischen und hyperosmolaren Konzentrationen von Salzen oder Salzgemischen, bevorzugt Kochsalz, insbesondere Meersalz.

Daher kann der mindestens eine flüssige Pflanzenextrakt in einer Zubereitung vorliegen, insbesondere in Form eines Arzneimittels oder Nahrungsergänzungsmittels, umfassend weitere Hilfs- und Zusatzstoffe.

### Beispiele:

Die folgenden Beispiele und Abbildungen sollen die Erfindung näher erläutern.

### Beispiel 1:

### Trübung der flüssigen Pflanzenextrakte und Ausfällungen

Eine Entwicklungscharge eines alkoholfreien Pflanzenextraktes aus fünf Pflanzen (Sambucus, Gentianae, Verbena, Rumex, Primula wie z.B. in Sinupret^{®} Saft) wurde nach der Herstellung in Flaschen mit und ohne Innensilikonisierung abgefüllt. Nach 6 Monaten Lagerzeit bei der Lagerbedingung 40°C/75% r. F. wurde die Trübung der zwei Proben verglichen. Die Probe, die in den Flaschen ohne Innensilikonisierung gelagert wurde, zeigt eine deutliche Trübung, die Lösung in der Flasche mit Innensilikonisierung ist weiterhin klar.

Ein Entwicklungsversuch mit anderer pflanzlicher Zusammensetzung(alkoholhaltiger Extrakt aus Thymus und Hedera wie z. B. in Bronchipret^{®} Tropfen) zeigt nach 12 Monaten Lagerung bei Raumtemperatur (RT) und 25°C/60% r. F. deutliche Ausfällungen im Packmittel ohne Innensilikonisierung). Der Niederschlag bildet eine gut erkennbare Schicht am Flaschenboden. In den Flaschen mit Innensilikonisierung ist ein Niederschlag nicht erkennbar.

### Beispiel 2:

### Veränderung der Zusammensetzung der Produkte

### pH-Wert

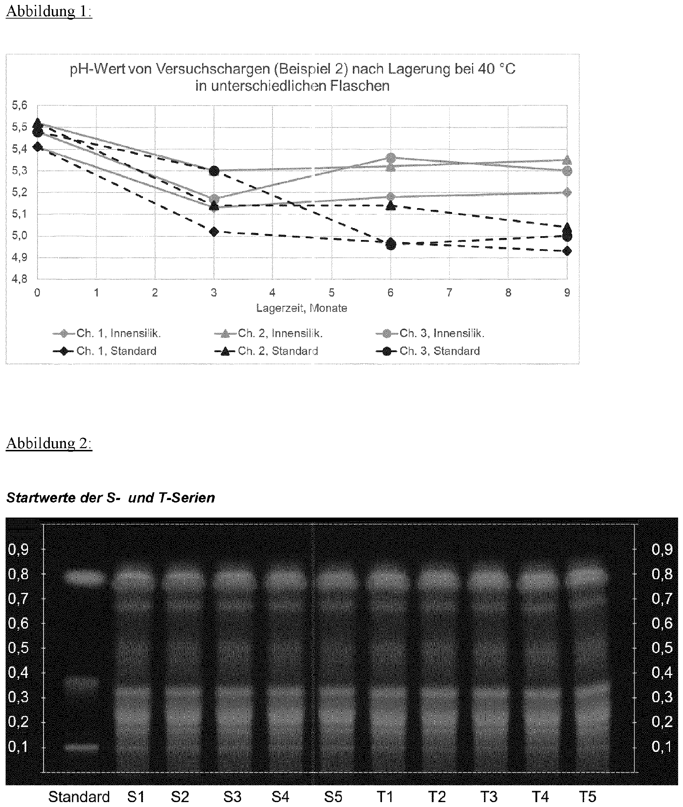

Der pH-Wert der Zubereitungen kann großen Einfluss auf die chemische Stabilität haben. Versuchschargen eines alkoholhaltigen Pflanzenextraktes aus fünf Pflanzen (Sambucus, Gentianae, Verbena, Rumex, Primula wie z. B. in Sinupret Tropfen) zeigen in einem Packmittel ohne Innensilikonisierung einen pH-Abfall. Die Veränderung der gleichen Chargen, in innen-silikonisierten Flaschen gelagert, ist kleiner (siehe Abb. 1).

### Chromatographische Fingerprints

Die Fingerprintchromatogramme der pharmazeutischen Versuche werden für die Bewertung der Haltbarkeit herangezogen. Dafür wird die Veränderung der Anzahl und der relativen Intensität der erfassten Zonen in Dünnschicht-Chromatogrammen mit dem Fingerprint aus der Ausgangsanalyse abgeglichen.

In den DC-Chromatogrammen einer Versuchsreihe der Produktfamilie (Bronchipret Saft und Tropfen) wurde die Auswirkung unterschiedlicher Primärpackmittel auf die Veränderung des Fingerprintes untersucht. Die Methode erfasst Polyphenole (z. B. Flavonoide).

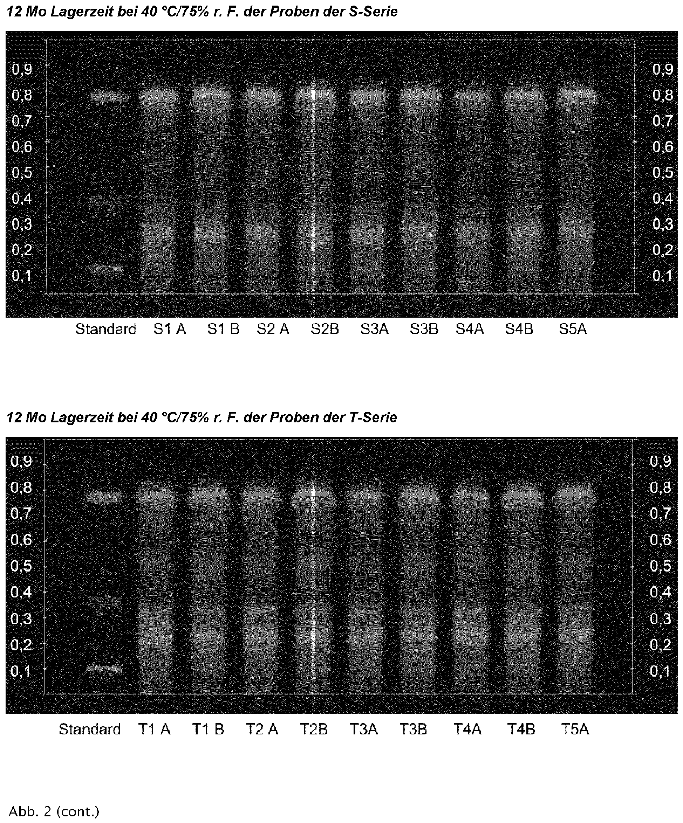

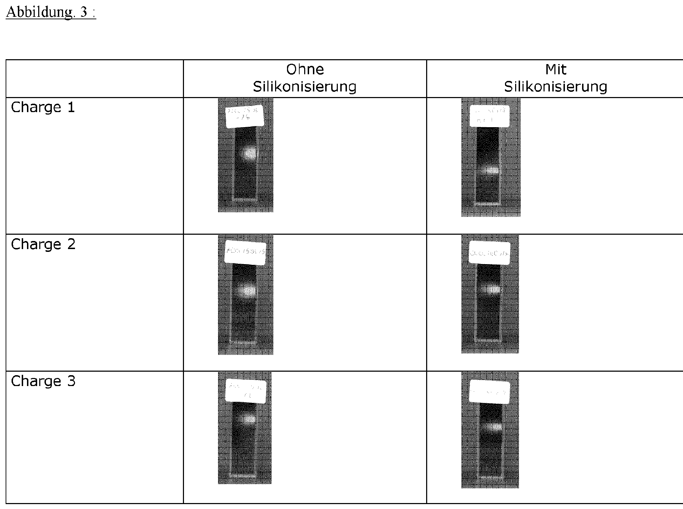

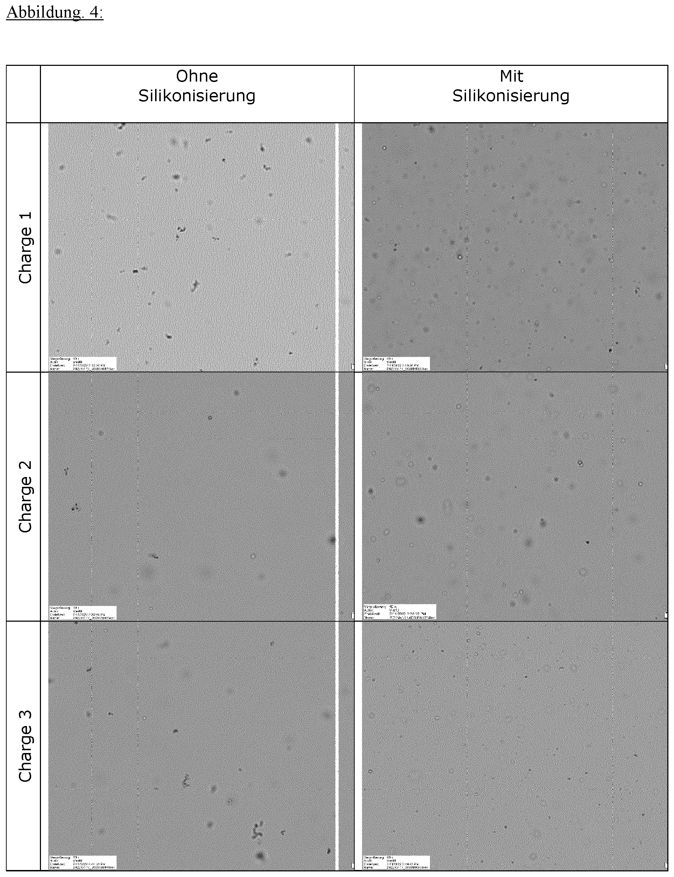

Jeweils 5 Produktvarianten (S- und T-Serie) wurden aus den gleichen Fluidextrakt-Chargen hergestellt. Die Varianten wurden unterschiedlich hergestellt (unterschiedlich filtriert) um die Auswirkung dieser Schritte auf die Haltbarkeit zu untersuchen. Alle Produktvarianten wurden in unterschiedlichen Flaschen abgefüllt und gelagert. Nach 12 Monaten Lagerzeit ist der Fingerprint der Proben, die in innen-silikonisierten Flaschen (B) gelagert waren, weniger verändert als in den nicht-innen-silikonisierten Flaschen (A) wie z.B. Glasart III (Abb. 2).

Besonders deutliche Unterschiede gibt es in beiden Serien bei den folgenden Zonen (R_{f}-Wert ist auf der y-Achse in Abb. 2 aufgetragen):

| | |
|---|---|
| R_{f} 0,1 | in innen-silikoniserten Flaschen (B) überall sichtbar, |
| R_{f} 0,2 | in innen-silikoniserten Flaschen (B) überall sichtbar, |
| R_{f} 0,7 | in innen-silikoniserten Flaschen (B) überall sichtbar, |
| R_{f} 0,8 | in innen-silikoniserten Flaschen (B) überall sichtbar. |

### Beispiel 3:

### Stabilisierung sekundärer Pflanzeninhaltsstoffe, insbesondere Polyphenole

Die durch die Oberflächenvergütung mittels Silikonisierung verbesserte Stabilität lässt sich auch deutlich anhand typischer Pflanzeninhaltsstoffe wie z.B. Phenolsäuren (Rosmarinsäure) oder Flavonoiden (z.B. Rutin) zeigen. Im direkten Vergleich zeigen Inhaltsstoffe einen signifikant höheren Abbau, wenn sie in nicht-innensilikonisiertem Glas gelagert werden. Rosmarinsäure baut im relativen Vergleich zu einer Lagerung in silikonisertem Glas um 40 % mehr ab, Apigening um 50 % mehr ab und Eriodictyol sogar fast komplett mit 91 % (vgl. Tabelle 1).

**Tabelle 1. Vergleichende Untersuchung des Abbaus typischer Inhaltsstoffe eines pflanzlichen Arzneimittels nach 12-monatiger Lagerung unter forcierten Bedingungen (40 °C und 75 % RF). Verglichen wird derselbe flüssige Pflanzenextrakt aus Chargen 1-3 , der einmal in innensilikonisierten Glasflaschen und einmal in nicht-innensilikonisierten Glasflaschen gelagert wurde.**

| | Relativer prozentualer Abbau von Inhaltsstoffen im nicht silikonisierten Glas im Vergleich zu innensilikonisiertem Glas (100 % jeweils) | | | | | |
|---|---|---|---|---|---|---|
| Relativer Abbau | Rosmarinsäure | Luteolin | Rutin | Naringenin | Apigenin | Eriodictyol |
| Charge 1 | 60 % | 75 % | 83 % | 91 % | 68 % | 92 % |
| Charge 2 | 40 % | 72 % | 76 % | 88 % | 68 % | 98 % |
| Charge 3 | 21 % | 54 % | 67 % | 83 % | 50 % | 82 % |
| Durchschnitt | 40 % | 67 % | 75 % | 87 % | 62 % | 91 % |

### Diskussion

Die Verwendung von innen-silikonisierten Glasbehältnissen erhöht die physikalische und chemische Stabilität von flüssigen Pflanzenextrakten die als flüssige pharmazeutische Zubereitungen in Verkehr gebracht werden.

Die Abbauprozesse von unterschiedlichen Inhaltsstoffen in flüssigen Pflanzenextrakten und deren Zubereitungen sind bei Verwendung von innen-silikonisierten Glasbehältnissen reduziert.

## Patentansprüche

1. Verwendung von einem verschließbaren, silikonisierten Glasbehälter zur Aufbewahrung von mindestens einem flüssigen Pflanzenextrakt, wobei der verschließbare, silikonisierte Glasbehälter eine Innenwand mit einer Silikonschicht aufweist, **dadurch gekennzeichnet, dass** der Pflanzenextrakt
i.) in einem oder mehreren Lösungsmitteln gelöst vorliegt, ausgewählt aus der Gruppe alkoholhaltige Lösungsmittel, C1-C5-Alkohole, Ethanol, Wasser, Gemisch aus Wasser / Ethanol , oder ein entalkoholisierter flüssiger Pflanzenextrakt davon und Polyphenole enthält,
ii.) wobei der Pflanzenextrakt ein Arzneimittel ist, und weitere Hilfs- und Zusatzstoffe aufweist,
iii.) wobei das Extraktionsmittel ausgewählt ist aus der Gruppe alkoholhaltige Lösungsmittel, C1-C5-Alkohole, Ethanol, Wasser, Gemisch aus Wasser / Ethanol.

2. Verwendung nach Anspruch 1, wobei der Pflanzenextrakt mittels Extraktion, Perkolation oder Mazeration aus Pflanzenmaterialien gewonnen wird oder ein Fluidextrakt ist.

3. Verwendung nach einem der Ansprüche 1 bis 2, wobei der verschließbare, silikonisierte Glasbehälter einseitig verschließbar ist, insbesondere durch einen Deckel, Aufsatz, Druckaufsatz oder Pumpapplikator.

4. Verwendung nach einem der Ansprüche 1 bis 3, wobei der verschließbare, silikonisierte Glasbehälter einen Drehverschluss aufweist.

5. Verwendung nach einem der Ansprüche 1 bis 4, wobei der verschließbare, silikonisierte Glasbehälter eine Innenwand mit einer Silikonschicht aufweist, welche durch Einbrennsilikonisierung oder Gasphasenabscheidung (CVD) hergestellt wird.

6. Verwendung nach einem der Ansprüche 1 bis 5, wobei die Aufbewahrung mindestens drei Monate, 6 Monate oder 12 Monate oder länger erfolgt.

7. Verwendung nach Anspruch 6, wobei keine Trübung oder Ausfällung erfolgt.

8. Verwendung nach einem der Ansprüche 1 bis 7, wobei der mindestens eine flüssige Pflanzenextrakt Polyphenole enthält, insbesondere Phenolsäuren, Phenolcarbonsäuren (z.B. Rosmarinsäure), Hydroxybenzoesäuren und Flavonoide.

9. Verwendung nach einem der Ansprüche 1 bis 8, wobei der mindestens eine flüssige Pflanzenextrakt aus einer Gattung ausgewählt ist aus der Gruppe Achillea, Aloe, Althaea, Angelica, Arnika, Artemisia, Cannabis, Capsicum, Carum, Caulophyllum, Centaurium, Chelidonium, Cimicifuga, Cnicus, Citrus, Crataegus, Cyclamen, Cynara, Echinacea, Equisetum, Glycyrrhiza, Guaiacum, Hedeara, Humulus, Iberis, Iris, Juglans, Lavandula, Levisticum, Lilium, Matricaria, Melissa, Mentha, Basilicum (Ocimum), Passiflora, Pelargonium, Phytolacca, Pimpinella, Primula, Potentilla, Punica, Quercus, Rosmarinus, Rumex, Salix, Salvia, Sambucus, Silybum, Strychnos, Taraxacum, Thymus, Vaccinium, Valeriana, Vebena, Vitex, Vitis.

10. Verwendung nach einem der Ansprüche 1 bis 9, wobei der mindestens eine Pflanzenextrakt aus einer Art ausgewählt ist aus der Gruppe Achillea millefolium Aloe Vera, Althaea officinalis, Angelica archangelica, Arnika montana, Artemisia vulgaris, annua und absinthium, Cannabis indica, Cannabis ruderalis, Cannabis sativa, Capsicum annuum, Carum carvi, Caulophyllum thalictroides, Centaurium, Chelidonium majus, Cimicifuga racemose, Cnicus benedictus Citrus reticulata, Citrus sinensis, Citrus junos, Citrus medica, Citrus maxima, Citrus aurantifolia, Citrus aurantium, Citrus hystrix, Citrus limon, Citrus paradisi, Cistus incanus, Crataegus, Cyclamen purpurascens, Cynara cardunculus, Echinacea purpurea und angust, Equisetum arvense, Glycyrrhiza glabra, Glycyrrhiza inflata, Glycyrrhiza uralensis, Guaiacum officinale und sanctum, Hedeara helix, Humulus lupulus, Iberis amara, Juglans regia, Lavandula angustifolia, Levisticum officinale, Lilium tigrinum, Matricaria chamomilla, Melissa officinalis, Mentha candensis, Mentha arvensis, Mentha piperita, Ocimum basilicum, Passiflora, Phytolacca americana, Pelargonium sidoides, Pimpinella anisum, Primula veris, elatior und vulgaris, Potentilla anserina, Punica granatum, Quercus robur, Quercus petraea, Quercus pubescens, Rosmarinus, Rumex cripus, Rumex obtusifolius, Rumex alpinus, Rumex patientia, Rumex acetosa, Rumex acetosella, Rumex thyrsiflorus, Salix purpurea, Salix daphnoides, Salix fragilis, Salvia officinalis, Sambucus nigra, Silybum marianum, Strychnos ignatii, Taraxacum officinale, Thymus vulgaris, Vaccinium macrocarpon, Vaccinium myrtillus, Valeriana officinalis, Vebena officinalis, Vitex agnus castus, Vitis vinifera.

11. Verwendung nach einem der Ansprüche 1 bis 10, wobei der mindestens eine flüssige Pflanzenextrakt in einer galenischen Form ausgewählt ist aus der Gruppe flüssiger Zubereitungen, Tropfen, Saft, Sirup, Aufguss, Rachenspray sowie Desinfektionslösungen, Nasenspray, flüssige Präparate für die Inhalation, Spüllösungen, insbesondere in Kombination mit physiologischen und hyperosmolaren Konzentrationen von Salzen oder Salzgemischen, bevorzugt Kochsalz, insbesondere Meersalz.

## Claims

1. Use of a closable, siliconized glass container for storing at least one liquid plant extract, the closable, siliconized glass container comprising an inner wall including a silicone layer,
**characterized in that** the plant extract
i.) is present dissolved in one or more solvents, selected from the group consisting of alcohol-containing solvents, C1 to C5 alcohols, ethanol, water, mixture of water/ethanol, or a dealcoholized liquid plant extract thereof and contains polyphenols,
ii.) the plant extract being a medicinal product and comprising further adjuvants and additives; and
iii.) the extracting agent being selected from the group of alcohol-containing solvents, C1 to C5 alcohols, ethanol, water, mixture of water/ethanol.

2. The use according to claim 1, wherein the plant extract is obtained from plant materials by means of extraction, percolation or maceration or is a fluid extract.

3. The use according to any one of claims 1 to 2, wherein the closable, silizonized glass container can be closed on one side, in particular by a lid, an attachment, a pressure attachment or a pump applicator.

4. The use according to any one of claims 1 to 3, wherein the closable, siliconized glass container comprises a twist top.

5. The use according to any one of claims 1 to 4, wherein the closable, siliconized glass container comprises an inner wall having a silicone layer, which is produced by way of bake-on siliconization or chemical vapor deposition (CVD).

6. The use according to any one of claims 1 to 5, wherein the storage takes place for at least three months, 6 months or 12 months or longer.

7. The use according to claim 6, wherein no turbidity or precipitation occurs.

8. The use according to any one of claims 1 to 7, wherein the at least one liquid plant extract contains polyphenols, in particular phenolic acids, phenolic carboxylic acids (for example rosmarinic acid), hydroxybenzoic acids, and flavonoids.

9. The use according to any one of claims 1 to 8, wherein the at least one liquid plant extract is selected from a genus selected from the group consisting of Achillea, Aloe, Althaea, Angelica, Arnica, Artemisia, Cannabis, Capsicum, Carum, Caulophyllum, Centaurium, Chelidonium, Cimicifuga, Cnicus, Citrus, Crataegus, Cyclamen, Cynara, Echinacea, Equisetum, Glycyrrhiza, Guaiacum, Hedeara, Humulus, Iberis, Iris, Juglans, Lavandula, Levisticum, Lilium, Matricaria, Melissa, Mentha, Basilicum (Ocimum), Passiflora, Pelargonium, Phytolacca, Pimpinella, Primula, Potentilla, Punica, Quercus, Rosmarinus, Rumex, Salix, Salvia, Sambucus, Silybum, Strychnos, Taraxacum, Thymus, Vaccinium, Valeriana, Vebena, Vitex, and Vitis.

10. The use according to any one of claims 1 to 9, wherein the at least one plant extract is selected from a species selected from the group consisting of Achillea millefolium Aloe Vera, Althaea officinalis, Angelica archangelica, Arnica montana, Artemisia vulgaris, annua and absinthium, Cannabis indica, Cannabis ruderalis, Cannabis sativa, Capsicum annuum, Carum carvi, Caulophyllum thalictroides, Centaurium, Chelidonium majus, Cimicifuga racemose, Cnicus benedictus, Citrus reticulata, Citrus sinensis, Citrus junos, Citrus medica, Citrus maxima, Citrus aurantifolia, Citrus aurantium, Citrus hystrix, Citrus limon, Citrus paradisi, Cistus incanus, Crataegus, Cyclamen purpurascens, Cynara cardunculus, Echinacea purpurea and angust, Equisetum arvense, Glycyrrhiza glabra, Glycyrrhiza inflata, Glycyrrhiza uralensis, Guaiacum officinale and sanctum, Hedeara helix, Humulus lupulus, Iberis amara, Juglans regia, Lavandula angustifolia, Levisticum officinale, Lilium tigrinum, Matricaria chamomilla, Melissa officinalis, Mentha candensis, Mentha arvensis, Mentha piperita, Ocimum basilicum, Passiflora, Phytolacca americana, Pelargonium sidoides, Pimpinella anisum, Primula veris, elatior and vulgaris, Potentilla anserina, Punica granatum, Quercus robur, Quercus petraea, Quercus pubescens, Rosmarinus, Rumex cripus, Rumex obtusifolius, Rumex alpinus, Rumex patientia, Rumex acetosa, Rumex acetosella, Rumex thyrsiflorus, Salix purpurea, Salix daphnoides, Salix fragilis, Salvia officinalis, Sambucus nigra, Silybum marianum, Strychnos ignatii, Taraxacum officinale, Thymus vulgaris, Vaccinium macrocarpon, Vaccinium myrtillus, Valeriana officinalis, Vebena officinalis, Vitex agnus castus, and Vitis vinifera.

11. The use according to any one of claims 1 to 10, wherein the at least one liquid plant extract in a galenic form is selected from the group consisting of liquid formulations, drops, juice, syrup, infusion, throat spray as well as disinfectant solutions, nasal spray, liquid preparations for inhalation, rinsing solutions, in particular in combination with physiological and hyperosmolar concentrations of salts or salt mixtures, preferably table salt, in particular sea salt.

## Revendications

1. Utilisation d'un récipient refermable en verre siliconé pour le stockage d'au moins un extrait de plante liquide, le récipient refermable en verre siliconé comprenant une paroi interne incluant une couche de silicone,
**caractérisé en ce que** l'extrait de plante
i.) est présent sous forme dissoute dans un ou plusieurs solvants, choisis dans le groupe constitué par les solvants alcooliques, les alcools en C1 à C5, l'éthanol, l'eau, un mélange eau/éthanol, ou un extrait de plante liquide désalcoolisé de celui-ci et contient des polyphénols,
ii.) l'extrait de plante étant un produit médicinal et comprenant des adjuvants et des additifs supplémentaires ; et
iii.) l'agent d'extraction étant choisi dans le groupe constitué par les solvants alcooliques, les alcools en C1 à C5, l'éthanol, l'eau, un mélange eau/éthanol.

2. Utilisation selon la revendication 1, dans laquelle l'extrait de plante est obtenu à partir de matériels de plantes par extraction, percolation ou macération ou est un extrait de fluide.

3. Utilisation selon l'une quelconque des revendications 1 à 2, dans laquelle le récipient refermable en verre siliconé peut être fermé sur un côté, en particulier par un couvercle, un module, un module de pression ou un adaptateur de pompe.

4. Utilisation selon l'une quelconque des revendications 1 à 3, dans laquelle le récipient refermable en verre siliconé comprend un bouchon à vis.

5. Utilisation selon l'une quelconque des revendications 1 à 4, dans laquelle le récipient refermable en verre siliconé comprend une paroi interne ayant une couche de silicone, qui est produite par siliconage par cuisson ou dépôt chimique en phase vapeur (CVD).

6. Utilisation selon l'une quelconque des revendications 1 à 5, dans laquelle le stockage se déroule pendant au moins trois mois, 6 mois ou 12 mois ou plus.

7. Utilisation selon la revendication 6, dans laquelle aucune turbidité ni précipitation ne surviennent.

8. Utilisation selon l'une quelconque des revendications 1 à 7, dans laquelle l'au moins un extrait de plante liquide contient des polyphénols, en particulier des acides phénoliques, des acides carboxylique phénoliques (par exemple l'acide rosmarinique), des acides hydroxybenzoïques et des flavonoïdes.

9. Utilisation selon l'une quelconque des revendications 1 à 8, dans laquelle l'au moins un extrait de plante liquide est choisi parmi un genre choisi dans le groupe constitué par Achillea, Aloe, Althaea, Angelica, Arnica, Artemisia, Cannabis, Capsicum, Carum, Caulophyllum, Centaurium, Chelidonium, Cimicifuga, Cnicus, Citrus, Crataegus, Cyclamen, Cynara, Echinacea, Equisetum, Glycyrrhiza, Guaiacum, Hedeara, Humulus, Iberis, Iris, Juglans, Lavandula, Levisticum, Lilium, Matricaria, Melissa, Mentha, Basilicum (Ocimum), Passiflora, Pelargonium, Phytolacca, Pimpinella, Primula, Potentilla, Punica, Quercus, Rosmarinus, Rumex, Salix, Salvia, Sambucus, Silybum, Strychnos, Taraxacum, Thymus, Vaccinium, Valeriana, Vebena, Vitex et Vitis.

10. Utilisation selon l'une quelconque des revendications 1 à 9, dans laquelle l'au moins un extrait de plante est choisi parmi une espèce choisie dans le groupe constitué par Achillea millefolium, Aloe Vera, Althaea officinalis, Angelica archangelica, Arnica montana, Artemisia vulgaris, annua et absinthium, Cannabis indica, Cannabis ruderalis, Cannabis sativa, Capsicum annuum, Carum carvi, Caulophyllum thalictroides, Centaurium, Chelidonium majus, Cimicifuga racemose, Cnicus benedictus, Citrus reticulata, Citrus sinensis, Citrus junos, Citrus medica, Citrus maxima, Citrus aurantifolia, Citrus aurantium, Citrus hystrix, Citrus limon, Citrus paradisi, Cistus incanus, Crataegus, Cyclamen purpurascens, Cynara cardunculus, Echinacea purpurea et angust, Equisetum arvense, Glycyrrhiza glabra, Glycyrrhiza inflata, Glycyrrhiza uralensis, Guaiacum officinale et sanctum, Hedeara helix, Humulus lupulus, Iberis amara, Juglans regia, Lavandula angustifolia, Levisticum officinale, Lilium tigrinum, Matricaria chamomilla, Melissa officinalis, Mentha candensis, Mentha arvensis, Mentha piperita, Ocimum basilicum, Passiflora, Phytolacca americana, Pelargonium sidoides, Pimpinella anisum, Primula veris, elatior et vulgaris, Potentilla anserina, Punica granatum, Quercus robur, Quercus petraea, Quercus pubescens, Rosmarinus, Rumex cripus, Rumex obtusifolius, Rumex alpinus, Rumex patientia, Rumex acetosa, Rumex acetosella, Rumex thyrsiflorus, Salix purpurea, Salix daphnoides, Salix fragilis, Salvia officinalis, Sambucus nigra, Silybum marianum, Strychnos ignatii, Taraxacum officinale, Thymus vulgaris, Vaccinium macrocarpon, Vaccinium myrtillus, Valeriana officinalis, Vebena officinalis, Vitex agnus castus et Vitis vinifera.

11. Utilisation selon l'une quelconque des revendications 1 à 10, dans laquelle l'au moins un extrait de plante liquide sous une forme galénique est choisi dans le groupe constitué par les formules liquides, les gouttes, un jus, un sirop, une infusion, un spray pour la gorge ainsi que des solutions désinfectantes, un spray nasal, des préparations liquides pour inhalation, des solutions de rinçage, en particulier en association avec des concentrations physiologiques et hyperosmolaires de sels ou de mélanges de sels, de préférence du sel de table, en particulier du sel de mer.
